# EUROPEAN PATENT SPECIFICATION

(11) **EP 2 118 063 B1**
(45) Date of publication and mention of the grant of the patent: **03.11.2010**
(21) Application number: 08700826.4
(22) Date of filing: 08.01.2008
(51) Int. Cl.: C07D 215/18, C07D 323/00

(54) **A METHOD FOR THE PREPARATION OF MONTELUKAST**
VERFAHREN ZUR HERSTELLUNG VON MONTEKULAST
PROCÉDÉ DE FABRICATION DE MONTÉLUKAST

(30) Priority: 09.01.2007 CZ 20070020
(43) Date of publication of application: 18.11.2009
(73) Proprietor: Zentiva, k.s., Dolni Mecholupy 102 37 Praha 10 (CZ)
(72) Inventor: HALAMA, Ales, 530 09 Pardubice (CZ); JIRMAN, Josef, 100 00 Praha 10 (CZ)
(74) Representative: Jirotkova, Ivana
(86) International application number: PCT/CZ2008/000002
(87) International publication number: WO 2008/083635

(56) References cited:
- WO-A-2005/105751
- NISHIKUBO T ET AL: "A NOVEL SYNTHESIS OF POLY(ESTER-ALT-SUFLIDE)S BY THE RING-OPENING ALTERNATING COPOLYMERIZATION OF OXIRANES WITH GAMMA- THIOBUTYROLACTONE USING QUATERNARY ONIUM SALTS OR CROWN ETHER COMPLEXES AS CATALYSTS" MACROMOLECULES, ACS, WASHINGTON, DC, US, vol. 31, no. 15, 28 July 1998 (1998-07-28), pages 4746-4752, XP000769973 ISSN: 0024-9297

## Description

### Technical Field

The invention deals with a new preparation process of Montelukast of formula I, i.e. a substance that is used to prepare medicaments for the treatment of asthma and allergies.

### Background Art

Montelukast, chemically [R-(E)]-1-[[[1-[3-[2-(7-chlor-2-quinolinyl)ethenyl]phenyl]-3-[2-(1-hydroxy-1-methylethyl)phenyl]propyl]thio]methyl]cyclopropaneacetic acid of formula I, is a well-known antiasthmatic and antiallergic drug. It is mainly the sodium salt of Montelukast described with formula II that is used in therapy.

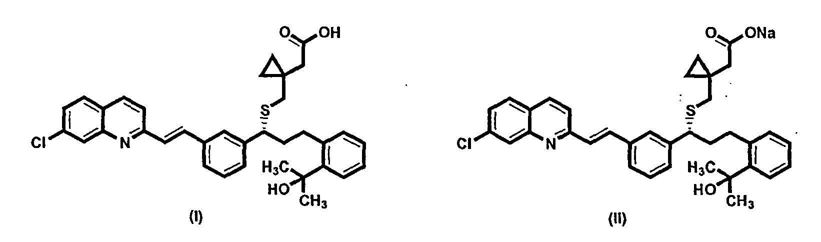

The sodium salt of Montelukast, its preparation and various forms, amorphous or crystalline, are described in a number of patents or applications, e.g. amorphous Montelukast Sodium is described in EP 0737186 B1 (MERCK, 1995), WO 03/066598 A1 (EDDY), WO 2004/108679 A1 (MOREPHEN, 2004), WO 2005/074893 A1 (CHEMAGIS). Crystalline polymorphs of Montelukast Sodium are dealt with by WO 2004/091618 A1 (MERCK), WO 2005/075427 A2 (TEVA).

The first method of chemical synthesis of Montelukast (I) was described in the patent no. EP 0480717 B1 (MERCK, 1992) and subsequently in specialized literature (M.Labele, Bioorg.Med.Chem.Lett. 5 (3), 283-288 (1995)). From the point of view of chemical synthesis of Montelukast of formula I the key step is binding of two building blocks (intermediates) with the use of a newly created bond between the carbon and sulphur atoms while the absolute configuration is maintained or completely inverted.

In principle, there are two basic methods of carrying out the key step of synthesis of Montelukast (I), which are indicated in Scheme 1. The first method represents formation of the C-S bond indicated as method A) in the diagram, the second one as method B). Both the basic methods can be further extended with a number of variants that are mainly based on alternating the order of reaction stages. The solution based on method A) is described in the following patents: EP 0480717 B1 (MERCK, 1992), EP 0737186 B1 (MERCK, 1995), US 2005/0234241 A1 (REDDY, 2005), WO 2005/105751 A1 (TEVA, 2005), US 2005/0107612 A1 (REDDY, 2005). The solution based on method B) is described in two patent applications of the SYNTHON Company: WO 2005/105749 A2 (SYNTHON, 2005), WO 2005/105750 A1 (SYNTHON, 2005).

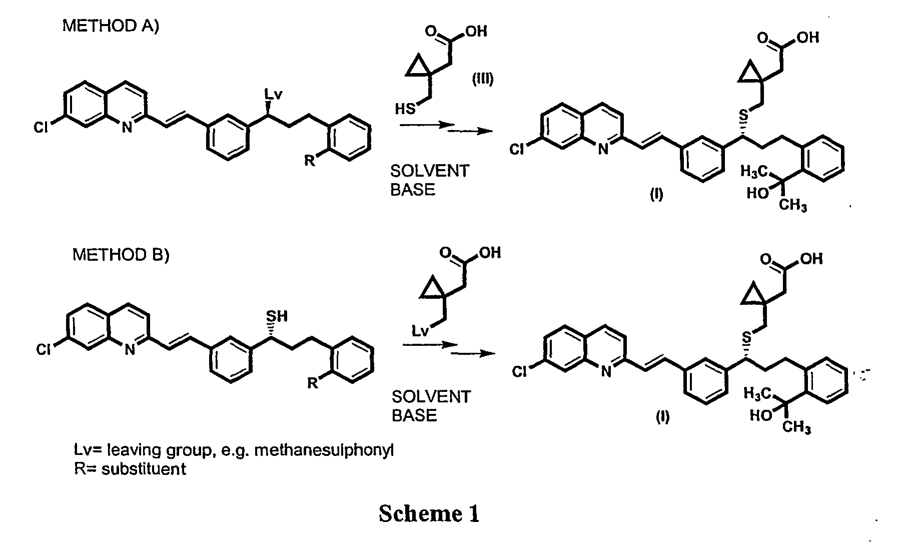

In practice mainly the solution is used that is characterized by the use of the dilithium salt of [1-(mercaptomethyl)cyclopropyl]acetic acid of formula III-diLi. The process is described in patent No. EP 0737186 B1 (MERCK, 1995); in an abbreviated form it is described in Scheme 2. This solution also comprises a method of purification of crude Montelukast of formula I via its salt with dicyclohexylamine and also a method of obtaining the sodium salt of Montelukast of formula II.

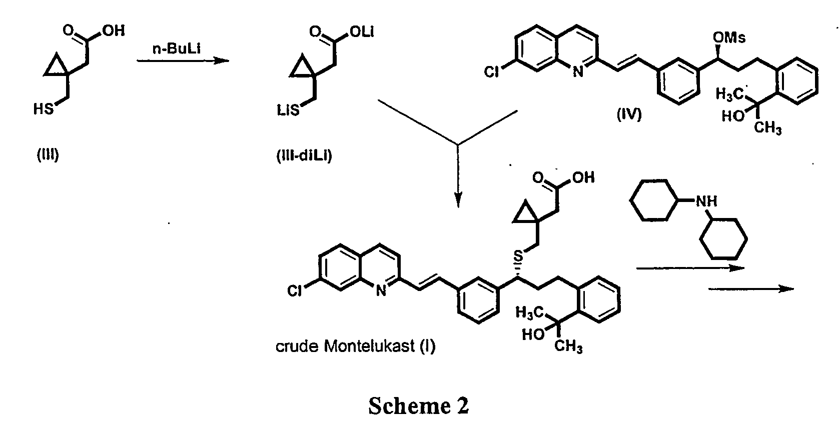

The process of preparation of Montelukast of formula I as well as the quality of the obtained product is affected by undesired reactions that both the starting materials and the product itself may be subject to depending on the process conditions. Literature describes increased sensitivity of the target substance to oxygen (see equation (1)) the substance of chemical formula (V) being described as the main product of oxidation of Montelukast of formula I (E.D. Nelson, J.Pharm.Sci. 95, 1527-1539 (2006), C. Dufresne, J.Org.Chem. 1996, 61, 8518-8525)). Contamination of the product with this or other impurities is most undesirable. For this reason processes of preparation of the target substance with the exclusion of oxygen are used, i.e. under the protective atmosphere of an inert gas (e.g. nitrogen in accordance with EP 0737186 B1).

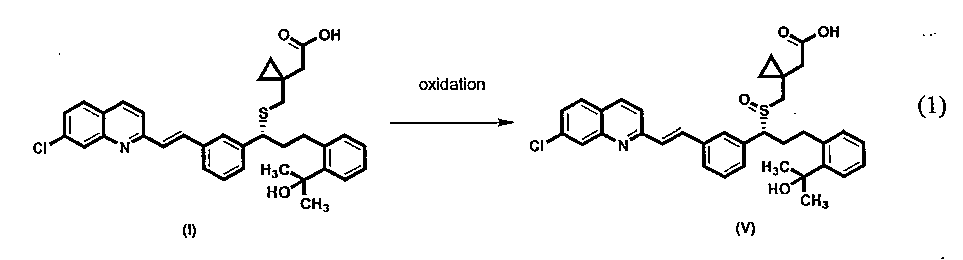

Another source of chemical contamination of Montelukast is instability of the normally used starting material described with formula IV. This substance is subject mainly to three unwanted reactions - hydrolysis, elimination and cyclization with the formation of impurities described with formulas (VI-VIII), see Scheme 3 (J.O. Egekeze, Anal.Chem. 1995, 67, 2292-2295).

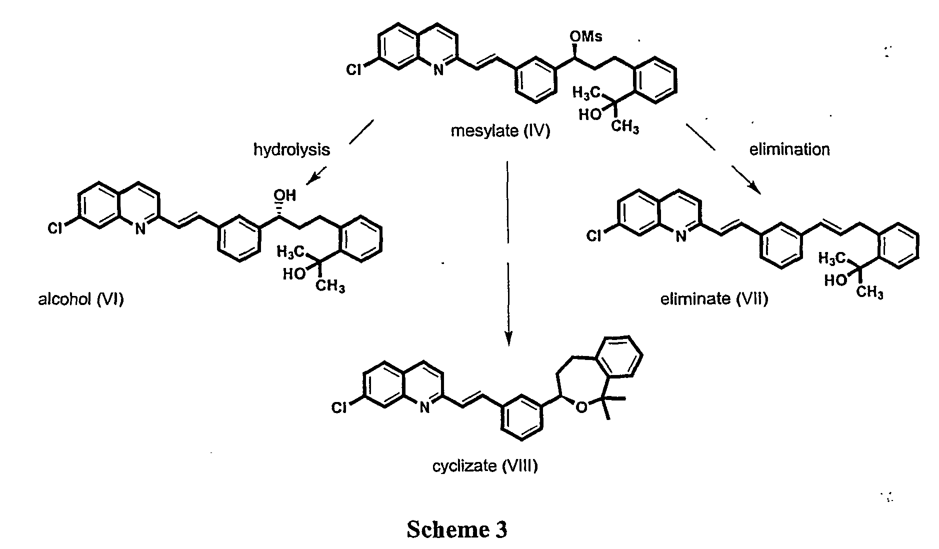

Hydrolysis can be prevented with the use of anhydrous organic solvents (e.g. THF in accordance with EP 0737186 B1), elimination can be prevented by performing the process at lower temperatures (below -5 °C in accordance with EP 0737186 B1), cyclization is prevented with the use of protective groups (e.g. tetrahydropyranyl (in short THP), M. Labele, Bioorg.Med.Chem.Lett. 5 (3), 283-288 (1995)), which however, increases the number of synthetic steps (introduction of the protective group and subsequently its deprotection), see Scheme 4.

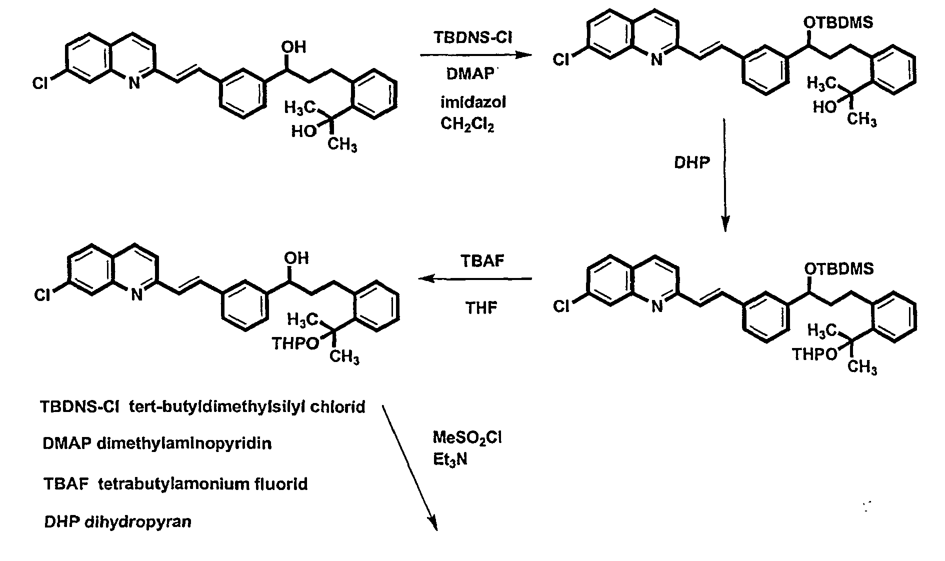

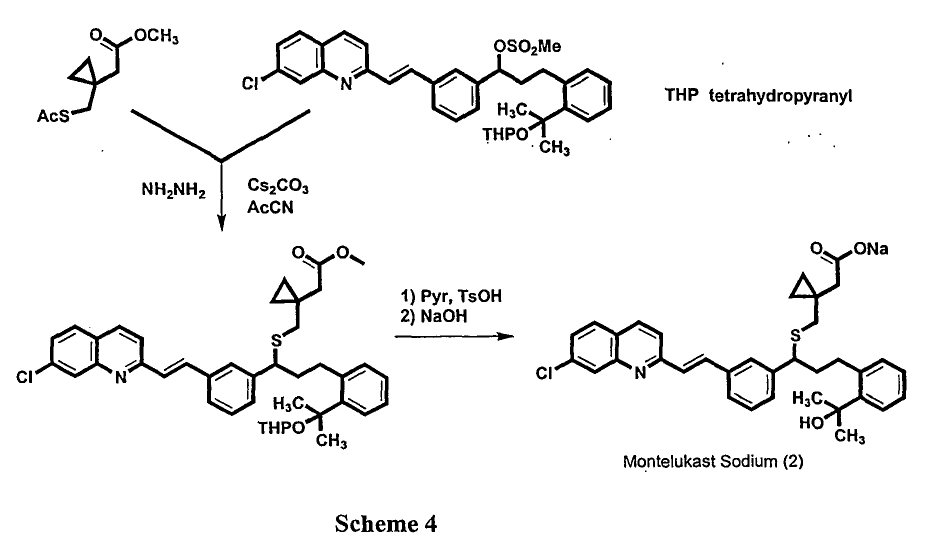

The present solution represents a new and advantageous method of carrying out the key substitution reaction that leads to Montelukast of formula I. The present procedure is distinguished from the previous solutions in the use of linear or cyclic polyethers, which ensure higher selectivity of the process and reduce the formation of undesired side products.

### Summary of the invention

The invention relates to a new method of carrying out the substitution reaction that represents the key stage in the process of preparation of Montelukast of formula I. This reaction is described by the following equation (2).

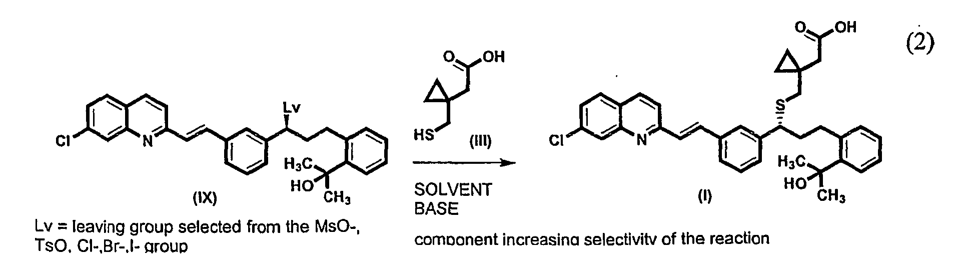

As the starting material of the present process of preparation of Montelukast of formula I a substance described with general formula IX can be used, preferably the substance of formula IV containing the methanesulphonic group as the leaving group. The starting material of formula IV is prepared e.g. in the way described in patents no. EP 0737186 B1 (MERCK, 1995), WO 2005/105751 A1 (TEVA, 2005) in accordance with the equation (3).

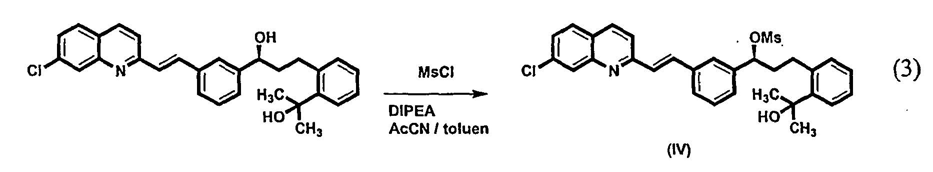

Another starting material of the present process is [1-(mercaptomethyl)cyclopropyl]acetic acid of formula III, which is converted to the corresponding salt by the effect of two base equivalents directly in the reaction mixture. This conversion is described by the equation (4). This salt only represents the active form of the reagent. Depending on the used ion the characteristics of these salts differ, mainly their solubility in the reaction environment. While the dilithium salt (III-diLi) is soluble for the most part in the used solvents, the disodium (III-diNa) and dipotassium (III-diK) salts do not dissolve for the greater part, which reduces the current concentration of the active agent in the solution. The resulting effect is deceleration of the required substitution reaction and reduction of its selectivity.

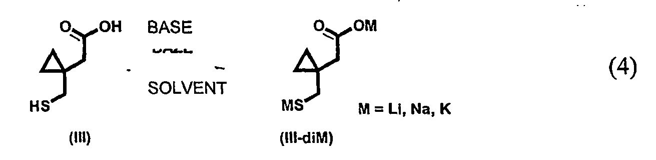

The process of the invention is further characterized with the use of bases that convert the acid of formula III to the respective salts (III-diM, where M indicates an alkali metal). As the base various substances can be used, e.g. organometallic compounds as well as alkali metal hydroxides and alkoxides of formula X. To ensure acceptable solubility suitable bases are alkoxides of metals, especially alkoxides characterized by a branched alkyl chain in their structure, e.g. tert-butoxides and tert-amylates of alkali metals (Na, K) described with formulas XI and XII.

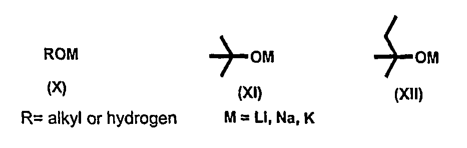

As the reaction environment inert organic solvents can be used, especially aromatic hydrocarbons, ethers, esters, amides, or sulphoxides or their mixtures in any proportions. E.g. the following solvents are suitable: toluene, benzene, tetrahydrofuran, methyltetrahydrofuran, dimethylcarbonate, dimethylformamide or dimethylsulphoxide. Using a mixture of toluene and tetrahydrofuran is particularly advantageous.

Unlike the previous solutions the present process makes use of advantageous properties of the polyethers of general formula XIII that can be either linear of formula XIV or cyclic of formulas XV-XVII. These substances play the role of phase transfer catalysts of complexing agents that solvate metal ions, increasing the solubility and reactivity of the used nucleophilic reagent, i.e. (III-diM). The increased reactivity of the reagent results in higher selectivity of the process, i.e. the impact of unwanted competitive reactions that lead to formation of impurities is suppressed. Thanks to the addition of the polyether to the reaction mixture the key synthetic stage occurs in a solution, not in the suspension phase.

As suitable linear polyethers polyethyleneglycols of formula XIV referred to with the PEG abbreviation can be used, mutually differing in their chain lengths or, more precisely, mixtures of polyethyleneglycols specified by the average molar weight, e.g. PEG-600 or PEG-1500. CROWN ethers of formulas XV-XVII with different cycle sizes can serve as suitable cyclic polyethers.

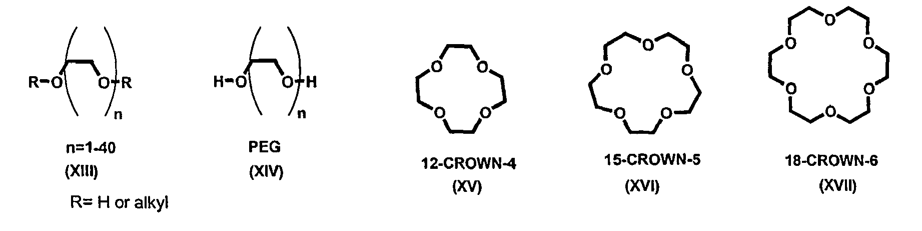

The reactions leading to the target substance of formula I were performed in accordance with the procedure of the present invention in such a way that first the carboxylic acid of formula III was mixed with the base of general formula X and polyether of formula XIII in the environment of an inert organic solvent and under an inert gas atmosphere. The obtained mixture was cooled down below -5 °C and then a solution of the substance of formula IV in a suitable organic solvent was added dropwise. Further, the reaction mixture was stirred under an inert atmosphere at the temperature of -5 to 40 °C for several hours and samples were drawn continuously to determine the conversion and selectivity of the substitution reaction. The crude product of formula I was finally isolated from the reaction environment with the yields of 65-75 % by commonly used processes that have been described before in patents nos. EP 0737186 B1**,** US 2005/0234241 A1**,** WO 2005/105751 A1**.**

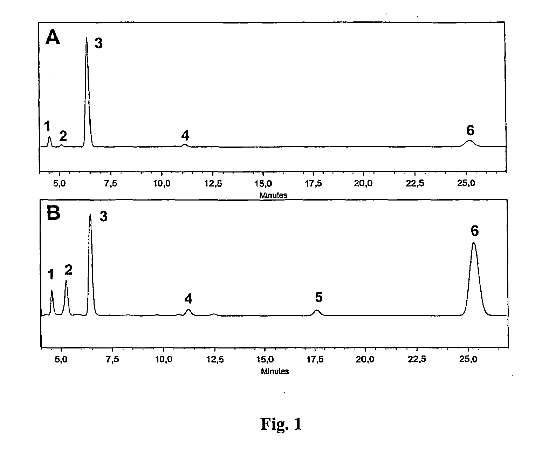

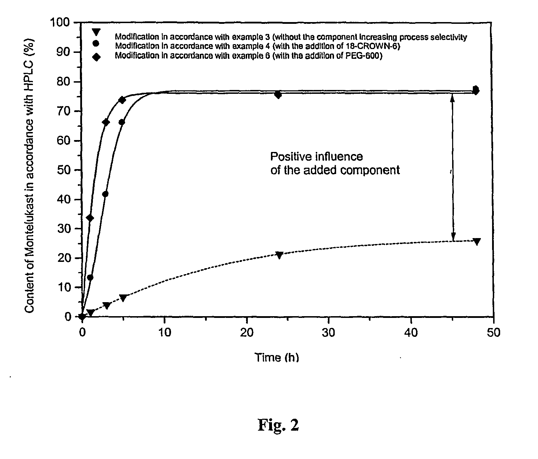

The process of the present invention is advantageous especially in that due to the addition of the polyether of formula XIII to the reaction mixture the desirable reaction (substitution) is accelerated as compared to undesired conversions (hydrolysis, elimination, cyclization). This way the negative influence of competitive reactions on the final composition of the reaction mixture at the end of the reaction, i.e. at the time of consumption of the starting substance, e.g. the substance of formula IV, is limited. Cyclic or linear polyethers of general formula XIII increase both solubility and reactivity (nucleophility) of the reagent (III-diM), which subsequently increases selectivity of the desired reaction. In carrying the key stage of preparation of Montelukast of formula I out in accordance with the present invention, the resulting composition of the reaction mixture is conveniently controlled without the necessity of long-term cooling or use of protective groups, which is a solution associated with an increase of the number of reaction stages. Besides the limitation of the content of impurities in the crude product our solution is also characterized by better utilization of the starting substances, i.e. the very expensive substance of formula IV in particular is not excessively consumed by undesired reactions. The advantageousness of our solution is demonstrated in the examples and also in figures 1 and 2 in the appendixes and in table 1. Figure 1 compares the composition of reaction mixtures of a reaction without the use of a cyclic or linear polyether and a reaction when these substances are added to the reaction mixture. Figure 2 compares the dependence of the contents of Montelukast in reaction mixtures on different conditions of the substitution reactions (in accordance with examples 3, 4 and 6). Table 1 compares conversions and selectivity measured after 24 hours from the mixing of the components in various modifications of the substitution reaction leading to Montelukast of formula I in accordance with examples 1-9.

### Brief Description of Drawings

Fig. 1 shows HPLC chromatograms of reaction mixtures of the substitution reaction of mesylate (IV)
   - **A** in accordance with example 4 after 48 hours (with the component increasing selectivity of the reaction: 18-CROWN-6).
   - **B** in accordance with example 3 after 48 hours (without addition of the component increasing selectivity of the reactions).
   Sequence of peaks: 1. alcohol (VI), 2. mesylate (IV), 3. Montelukast (I), 4. eliminate (VII), 5. unknown impurity, 6. cyclizate (VIII).
Fig. 2 shows the dependence of contents of Montelukast in the reaction mixture in substitution reactions carried out under various conditions (in accordance with examples 3, 4 and 6).

### Examples

The subject matter of the invention will be explained in a more detailed way with the use of the following examples, which however, do not have any influence on the scope of the invention defined in the claims.

### EXAMPLE 1 (Reference example)

In 20 ml of toluene [1-(mercaptomethyl)cyclopropyl]acetic acid (0.28 g) and the base (lithium tert-butoxide, 0.31 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 2 (Reference example)

In 20 ml of toluene [1-(mercaptomethyl)cyclopropyl]acetic acid (0.28 g), the base (lithium tert-butoxide, 0.31 g) and 12-crown-4 (0.33 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 3 (Reference example)

In 20 ml of toluene [1-(mercaptomethyl)cyclopropyl]acetic acid (0.28 g) and the base (sodium tert-butoxide, 0.36 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 4

In 20 ml of toluene [1-(mercaptomethyl)cyclopropyl]acetic acid (0.28 g), the base (sodium tert-butoxide, 0.36 g) and 18-crown-6 (0.50 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 5

In 20 ml of toluene [1-(mercaptomethyl)cyclopropyl]acetic acid (0.28 g), the base (sodium tert-butoxide, 0.36 g) and 15-crown-5 (0.41 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 6

In 20 ml of toluene [1-(mercaptomethyl)cyclopropyl]acetic acid (0.28 g), the base (sodium tert-butoxide, 0.36 g) and PEG-600 (1.1 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 7

In 20 ml of toluene [1-(mercaptomethyl)cyclopropyl]acetic acid (0.28 g), the base (sodium tert-butoxide, 0.36 g) and PEG-1500 (2.7 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 8 (Reference example)

In 20 ml of toluene [1-(mercaptomethyl)cyclopropyl]acetic acid (0.28 g) and the base (potassium tert-amylate, 0.42 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

### EXAMPLE 9

In 20 ml of toluene [1-(mercaptomethyl)cyclopropyl]acetic acid (0.28 g), the base (potassium tert-amylate, 0.42 g) and 18-crown-6 (0.50 g) were mixed and the mixture wan stirred in argon atmosphere and cooled to ca. -10 °C. A solution of 2-(3-(S)-(3-(2-(7-chloroquinolinyl)-ethenyl)phenyl)-3-methanesulphonyloxypropyl)phenyl-2-propanol (1 g) in 5 ml of tetrahydrofuran was subsequently added to the obtained slurry. The reaction mixture was stirred gradually from -10 °C up to the laboratory temperature for an hour. Then it was stirred at the laboratory temperature (about 21 °C) for several hours. The reaction mixture was continuously analyzed with HPLC.

**Table 1. Comparing conversions and selectivities after 24 hours from mixing of the components in different modifications of the substitution reaction leading to Montelukast (I)**

| Sample no. | Component increasing reaction selectivity | Conversion (%) | Selectivity (%) |
|---|---|---|---|
| 1 | ----- | 97.9 | 60.8 |
| 2 | 12-CROWN-4 | 97.3 | 59.6 |
| 3 | ----- | 82.3 | 26.0 |
| 4 | 18-CROWN-6 | 96.1 | 79.0 |
| 5 | 15-CROWN-5 | 94.6 | 77.5 |
| 6 | PEG-600 | 93.2 | 81.1 |
| 7 | PEG-1500 | 81.4 | 68.2 |
| 8 | ----- | 95.0 | 9.6 |
| 9 | 18-CROWN-6 | 92.7 | 51.5 |

### DESCRIPTION OF THE ANALYTICAL METHOD

Conversions and selectivities in the present process of preparation of Montelukast were determined with the use of the HLPC method. The chromatograms were measured with the EliteLachrom device made by the Hitachi Company. As the mobile phase a mixture of acetonitrile (80%) and of a 0.1M aqueous solution of ammonium formate adjusted to pH 3.6 with formic acid (20%). The measurements were performed in an isocratic mode with the flow rate of the mobile phase of 1.5 ml/min.

## Claims

1. A method of preparation of Montelukast of formula I by reaction of the compound of formula III with a compound of formula IX, 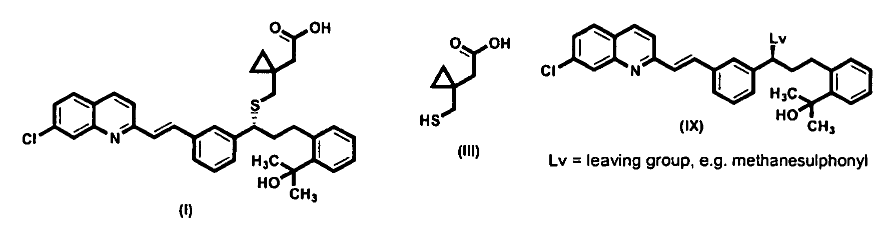 **characterized in that** the reaction is carried out in the presence of a base, an inert solvent and a component increasing selectivity of the process, wherein as the component increasing process selectivity a polyether of general formula XIII 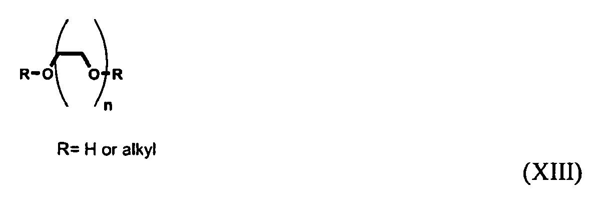 wherein R stands for hydrogen or an alkyl and the value of n varies from 1 to 40, or
a CROWN ether described with the formula (XV), (XVI) or (XVII) 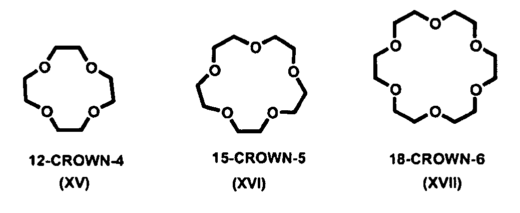 is used,
and
and as the said base an alkali metal alkoxide described with the general formula R'OM is used, wherein R' stands for an alkyl and M stands for an alkali metal selected from the group consisting of Na or K.

2. The method according to claim 1, **characterized in that** as the component increasing process selectivity a polyethyleneglycol of general formula XIV is used, where n=1 to 40.

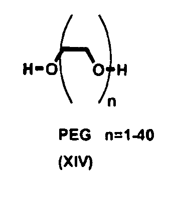

3. The method according to claim 1, **characterized in that** as the starting substance a substance of formula (IV) is used, wherein Ms stands for the methanesulphonyl leaving group.

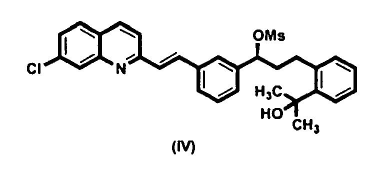

4. The method according to claim 1, **characterized in that** as the base an alkali metal alkoxide described with the general formula (XI) or (XII) is used, wherein M stands for an alkali metal selected from the group consisting of Na and K.

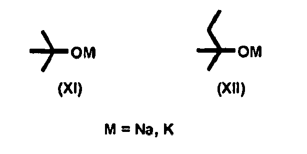

5. The method according to any of claims 1 to 4, **characterized in that** as the inert organic solvent an aromatic hydrocarbon, ether, ester, amide or sulphoxide, or their mixtures in any proportions are used.

6. The method according to claim 5, **characterized in that** as the inert organic solvent toluene, benzene, tetrahydrofuran, 2-methyltetrahydrofuran, dimethylcarbonate, dimethylformamide or dimethylsulphoxide or their mixtures in any proportions are used.

7. The method according to claim 6, **characterized in that** as the inert organic solvent a mixture of toluene and tetrahydrofuran is used.

8. The method according to any of claims 1 to 7, **characterized in that** first the carboxylic acid of formula (III) is mixed with a base of the general formula (X) and a polyether of formula (XIII) in the environment of an inert organic solvent and under an inert gas atmosphere, the obtained mixture is subsequently cooled and a solution of the substance (IV) in an organic solvent is added dropwise, the reaction mixture being further stirred under the inert gas atmosphere until the consumption of the starting substances.

9. The method according to claim 8, **characterized in that** the reacting components are mixed under cooling down, preferably below -5 °C, and the reaction is subsequently continued at the temperatures of the reaction mixture from -5 to +40 °C, preferably at the laboratory temperature of about 20 to 25 °C.

## Patentansprüche

1. Verfahren zur Herstellung von Montelukast der Formel I durch Umsetzen der Verbindung der Formel III mit einer Verbindung der Formel IX, 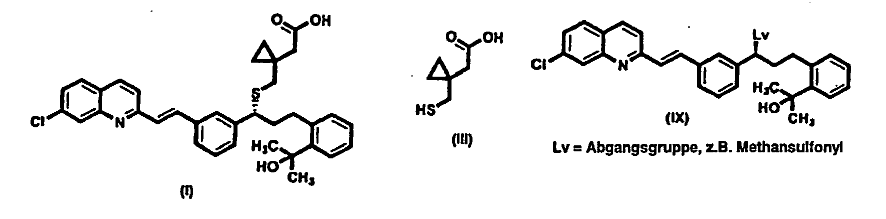 **dadurch gekennzeichnet, dass** die Umsetzung in Gegenwart einer Base, eines inerten Lösungsmittels und einer Komponente erfolgt, welche die Selektivität des Prozesses erhöht, wobei als die die Prozessselektivität erhöhende Komponente ein Polyether der allgemeinen Formel XIII 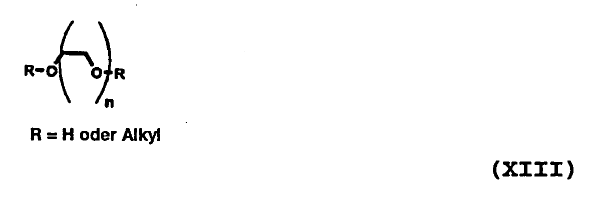 worin R für Wasserstoff oder ein Alkyl steht und der Wert von n in einem Bereich von 1 bis 40 variiert,
oder
ein Kronenether, dargestellt durch die Formel (XV), (XVI) oder (XVII) 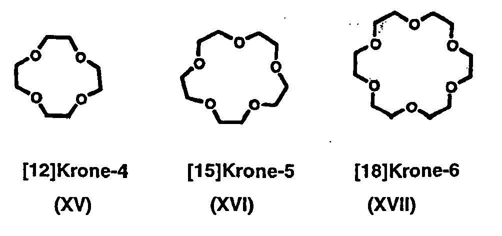 verwendet wird
und
als die Base ein Alkalimetallalkoxid, dargestellt durch die allgemeine Formel R'OM, verwendet wird, worin R' für ein Alkyl und M für ein Alkalimetall steht, das aus der Gruppe, die aus Na oder K besteht, ausgewählt ist.

2. Verfahren nach Anspruch 1, **dadurch gekennzeichnet, dass** als die die Prozessselektivität erhöhende Komponente ein Polyethylenglykol der allgemeinen Formel XIV verwendet wird, worin n = 1 bis 40.

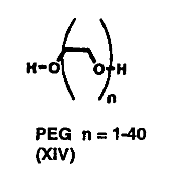

3. Verfahren nach Anspruch 1, **dadurch gekennzeichnet, dass** als der Ausgangsstoff ein Stoff der Formel (IV) verwendet wird, worin Ms für die Abgangsgruppe Methansulfonyl steht.

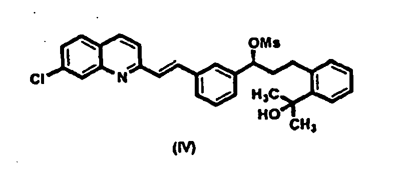

4. Verfahren nach Anspruch 1, **dadurch gekennzeichnet, dass** als Base ein Alkalimetallalkoxid, dargestellt durch die allgemeine Formel (XI) oder (XII), verwendet wird, in welcher M für ein Alkalimetall steht, das aus der Gruppe, die aus Na und K besteht, ausgewählt ist.

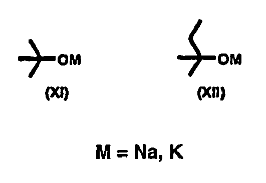

5. Verfahren nach einem oder mehreren der Ansprüche 1 bis 4, **dadurch gekennzeichnet, dass** als das inerte organische Lösungsmittel ein aromatischer Kohlenwasserstoff, Ether, Ester, Amid oder Sulfoxid, oder Mischungen beliebiger Anteile dieser Stoffe, verwendet werden.

6. Verfahren nach Anspruch 5, **dadurch gekennzeichnet, dass** als das inerte organische Lösungsmittel Toluol, Benzol, Tetrahydrofuran, 2-Methyltetrahydrofuran, Dimethylcarbonat, Dimethylformamid oder Dimethylsulfoxid, oder Mischungen beliebiger Anteile dieser Stoffe, verwendet werden.

7. Verfahren nach Anspruch 6, **dadurch gekennzeichnet, dass** als das inerte organische Lösungsmittel eine Mischung aus Toluol und Tetrahydrofuran verwendet wird.

8. Verfahren nach einem oder mehreren der Ansprüche 1 bis 7, **dadurch gekennzeichnet, dass** zunächst die Carbonsäure der Formel (III) mit einer Base der allgemeinen Formel (X) und einem Polyether der Formel (XIII) in der Umgebung eines inerten organischen Lösungsmittels und unter InertgasAtmosphäre gemischt wird, die erhaltene Mischung anschließend abgekühlt und eine Lösung des Stoffes (IV) in einem organischen Lösungsmittel tropfenweise hinzugegeben wird, wobei das Reaktionsgemisch solange unter der InertgasAtmosphäre weitergerührt wird, bis die Ausgangsstoffe verbraucht sind.

9. Verfahren nach Anspruch 8, **dadurch gekennzeichnet, dass** die Mischung der Reaktionskomponenten unter Kühlung, vorzugsweise auf unter -5°C, stattfindet und die Reaktion anschließend bei den Temperaturen des Reaktionsgemisches von -5 bis +40 °C fortgeführt wird, vorzugsweise bei der Labortemperatur von etwa 20 bis 25 °C.

## Revendications

1. Un procédé de préparation de Montelukast de formule I par réaction
du composé de formule III avec un composé de formule IX, 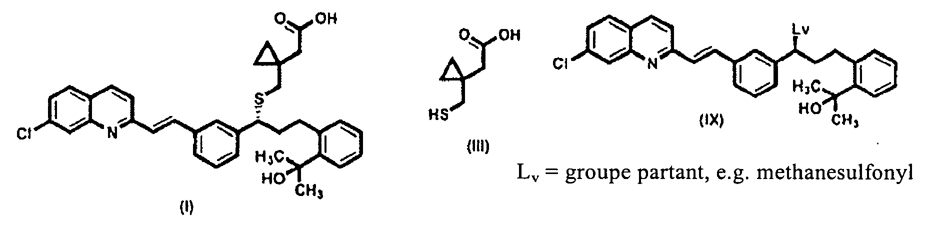 **caractérisé en ce que** la réaction est effectuée en présence d'une base, d'un solvant inerte et d'un composé augmentant le caractère sélectif du procédé, procédé dans lequel en tant que composé augmentant le caractère sélectif du procédé est employé un polyéther de formule générale XIII 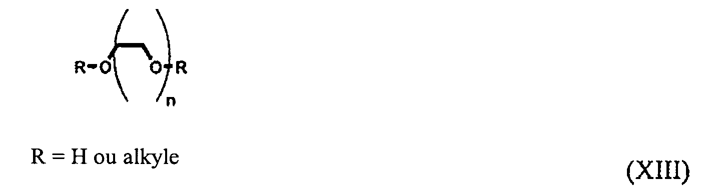 formule dans laquelle R est un atome d'hydrogène ou un radical alkyl et la valeur de n varie de 1 à 40,
ou est employé un éther couronne représenté par la formule (XV), (XVI) ou (XVII) 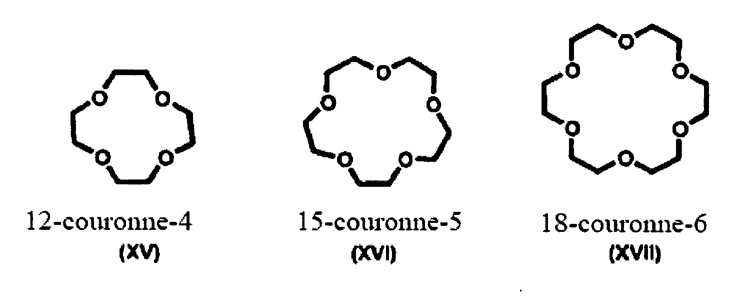 et en tant que ladite base est employé un alkoxyde d'un métal alcalin tel que représenté par la formule générale R'OM, où R' est un radical alkyle et M est un métal alcalin consistant en Na ou K.

2. Le procédé selon la revendication 1, **caractérisé en ce que** en tant que composé augmentant le caractère sélectif du procédé est employé un polyéthylèneglycol de formule générale XIV où n=1 à 40.

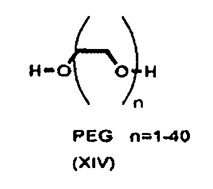

3. Le procédé selon la revendication 1, **caractérisé en ce que** en tant que matière de départ est employé un composé de formule (IV), procédé dans lequel Ms consiste en un groupe méthanesulfonyl partant.

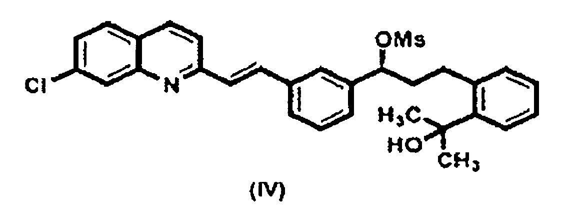

4. Le procédé selon la revendication 1, **caractérisé en ce que** en tant que base est employé un alkoxyde d'un métal alcalin représenté par la formule générale (XI) ou (XII), procédé dans lequel M est un métal alcalin consistant en Na ou K.

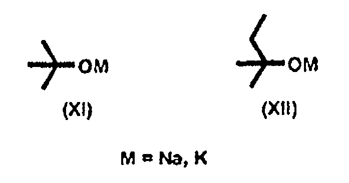

5. Le procédé selon une quelconque des revendications 1 à 4, **caractérisé en ce que** en tant que solvant organique inerte est employé un hydrocarbure aromatique, un éther, un ester, un amide ou un sulfoxyde, ou leurs mélanges en n'importe quelle proportion.

6. Le procédé selon la revendication 5, **caractérisé en ce qu'**en tant que solvant organique inerte est employé toluène, benzène, tétrahydrofuranne, 2-méthyltétra-hydrofurane, diméthyl carbonate, diméthylformamide ou diméthylsulfoxyde ou leurs mélanges en n'importe quelle proportion.

7. Le procédé selon la revendication 6, **caractérisé en ce que** en tant que solvant organique inerte est employé un mélange de toluène et de tétrahydrofuranne.

8. Le procédé selon une quelconque des revendications 1 à 7, **caractérisé en ce que** tout d'abord l'acide carboxylique de formule (III) est mélangé avec une base de formule générale (X) et un polyéther de formule (XIII), dans un milieu consistant en un solvant organique inerte et sous une atmosphère d'un gaz inerte, le mélange obtenu étant ensuite refroidi puis additionné, goutte à goutte, d'une solution du composé (IV) dans un solvant organique, le mélange réactionnel subissant alors une agitation supplémentaire dans une atmosphère de gaz inerte jusqu'à consommation de toutes les matières de départ.

9. Le procédé selon la revendication 8, **caractérisé en ce que** les composés de la réaction sont mélangés sous refroidissement, de préférence à une température inférieure à -5°C, et la réaction se poursuit alors à une température où le milieu réactionnel est maintenu à une température de -5 à +40 °C, de préférence à la température régnant dans un laboratoire de l'ordre d'environ 20 à 25°C.
